# EUROPEAN PATENT APPLICATION

(11) **EP 1 721 603 A1**
(43) Date of publication of application: **15.11.2006**
(21) Application number: 05010184.9
(22) Date of filing: 11.05.2005
(51) Int. Cl.: A61K 9/14

(54) **Nanoparticles for bioconjugation**

(71) Applicant: Albert-Ludwigs-Universität Freiburg, 79085 Freiburg (DE)
(72) Inventor: Riegler, Jürgen, 79117 Freiburg (DE); Nann, Thomas, 79112 Freiburg (DE)

(57) **Abstract**

The invention relates to a method for derivatising nanoparticles so that they can be obtained as dry preparations, which can be coupled to practically any biologically interesting component, (e.g. biomolecule or biologically active molecule, in any solvent; to said derivatised nanoparticles and their use, e.g. in the fields of analytics, diagnostics and/or therapy.

## Description

### Field of the Invention

The invention relates to a method for derivatising nanoparticles so that they can be obtained as dry preparations, which can be coupled to practically biologically interesting component in any solvent; to said derivatised nanoparticles and their use, e.g. in the fields of analytics, diagnostics and/or therapy.

### Background of the invention (Stand der Forschung)

This invention relates to the field of the utilisation of nanoparticles, preferable in the lower nanometer size regime, in biological applications, especially as probes for biomolecules and the production of biosensors. The invention also relates to the field of methods of manufacturing nanoparticle-biomolecule conjugates by ligand exchange and subsequent cross-linking of the ligands and coupling to biologically interesting components, e.g. proteins, peptides or nucleotides.

Nanoparticles in the lower nanometer size regime usually show a combination of properties both of their bulk counterparts and of soluble molecules. This makes them interesting as probes in bioanalytical and medical applications. Examples for beneficial optical properties are the size-dependent, stable photoluminescence of semiconductor nanocrystals (so called Quantum Dots), up-conversion luminescence in rare-earth doped nanoparticles (e.g. NaYF₄:Er,Yb) or afterglow in doped nanocrystals like e.g. ZnS:Cu,Co,Pb. Other desirable properties are superparamagnetism for magnetic resonance imaging (MRI), photostimulated luminescence (PSL) or contrast improving nanoparticles for optical coherence tomography (OCT). Both, the variability and multitude of different possible detection techniques and the reliability of the tags with nanoparticles are highly advantageous over the frequently used organic labels.

A major drawback for the application of nanoparticles as biological probes, however, up to now resides in the fact that nanoparticles, in their property as colloids, tend to coagulate in solutions of higher ionic strength. It was tried to overcome this problem by various approaches. For example, Quantum Dots (QD) were derivatised with chemisorbed monolayers (Chan WCW et al., 1998 Science, 281, pages 2016-2018), silica layers (Bruchez M Jr et al., 1998 Science, 281, pages 2013-2016) or phospholipid micelles (Dubertret et al., 2002 Science, 298, pages 1759-1762). Most of these strategies led to some success with regard to certain (specific) applications. However, a reliable, generally applicable method for coupling nanoparticles to biomolecules, leading to products which are dispersible in almost any aqueous buffer, is still lacking.

Nevertheless, nanoparticles for bioanalytical probing are already commercially available. Examples are CdSe/ZnS nanocrystals sold by Quantum Dot Corp. (QDC), Hayward, CA, USA or Fe₃O₄ nanoparticles (Resovist®) by Schering GmbH, Berlin, Germany. All of the known commercial products are non-specific, comparatively large and/or coagulate under physiological conditions.

The major problem to be solved by the present invention is to avoid the mentioned limitations and especially to provide nanoparticles in the lower nanometer size regime appropriate for conjugation with practically all kinds of blomolecules in order to allow for tagging biomolecules with, for example, luminescent nanoparticles. It is also a goal of the invention to provide nanoparticles that can be stored and traded conveniently and that are dispersible in all common buffer systems appropriate for biological applications. Although there are many previous publications describing procedures to couple nanoparticles to proteins and nucleotides, these procedu es are only applicable under special conditions and no general method exists so far. Thus, another problem to be solved by the present invention is to find a method to more generally label blomolecules with many different types of nanoparticles.

### Summary of the invention

In summary, the invention is based on a novel method for derivatising nanoparticles in such a way that, surprisingly, they are obtainable as dry preparations which can be used for coupling to almost any biornolecule in any solvent and especially have the advantage that they are very stable and thus can be stored for longer periods of time. The invention relates to these novel nanoparticles, a process for their manufacture and/or their use. The invented surface derivatisation method can be applied to almost any type of nanoparticles.

### Detailed description of the invention

In one general embodiment, the invention relates to a process for the manufacture of nanoparticles clad with an appropriate coating material.

In a first embodiment, the invention relates to a method for the manufacture of nanoparticles carrying a first type of coating with hydroxyl groups that are accessible from the outside of said nanoparticles, comprising reacting nanoparticles exposing hydroxyl, mercapto or amino groups accessible from the outside of the nanoparticles carrying said coating with a cross-linking reagent that is capable of cross-linking said hydroxy, mercapto or amino groups and itself carries hydroxy groups or precursor groups thereof and, if required, setting free the hydroxy groups from their precursor groups.

A cross-linking reagent that is capable of cross-linking said hydroxy, mercapto or amino groups and itself carries hydroxy groups or precursor groups thereof and, if required, setting free the hydroxy groups from their precursor groups is preferably a silazane or more preferably a silane carrying three or more Si-bound halo, e.g. chloro or bromo, and/or especially alkyloxy, especially C₁-C₇-alkoxy, such as methoxy or ethoxy, groups. A preferred example is tetra-(C₁-C₇-alkoxy)-silane, such as tetra-(ethoxy)-silane (TEOS).

The reaction preferably takes place in customary solvents, especially in alcohols, such as hydroxy-C₁-C₇-alkanes, especially corresponding to the respective moieties in the preferred tetra-(C₁-C₇-alkoxy)-silane crosslinkers, such as ethanol.

Preferably, the reaction takes place in the presence of a nitrogen base catalyst with at least one active hydrogen at the nitrogen, such as ammonia, and/or with lower preference primary or secondary amines, such as mono- or di-(C₁-C₇-alkyl)-amines.

The possible temperatures for the reaction are from -80 °C to the reflux temperature of the reaction mixture, for example preferably in the range from 0 to 50 °C, e.g. from 4 °C to 30 °C The setting free the hydroxy groups from their precursor groups preferably takes place by hydrolysis; preferably, the amount of water present in the reaction mixture is sufficient to lead to this hydrolysis, so that the crosslinking and the hydrolysis take place in parallel.

Preferably, in an additional process step the nanoparticles with the first type of coating are then isolated, e.g. by filtration, sedimentation or preferably precipitation and optionally washing with a solvent of lower polarity, such as a di-C₁-C₇-alkyl ether, e.g. diethylether, or an alcohol, e.g. ethanol, and dried to form a dry preparation, especially in the form of an amorphous powdery material, e.g. under vacuum, especially by lyophilisation, most preferably forming an amorphous lyophilisate. This nanoparticle preparation, especially the lyophilisate, is readily dispersible in polar media, such as water, buffer solutions and the like.

The first type of coating may fully cover each nanoparticle or may be interrupted, e.g. having pinholes. Preferred is a more or less complete coating in order to provide a maximum of hydroxy groups available for later coupling with e.g. biomolecules.

The nanoparticles exposing hydroxyl, mercapto or amino groups accessible from the outside of the nanoparticles carrying said coating used as starting materials are preferably silica-coated nanoparticles (which carry silanol hydroxyl groups) or nanoparticles obtainable, especially obtained, by the process described as second embodiment of the invention.

Both in the process of the first and the second embodiment of the invention as well as throughout the specification, the term "nanoparticle", if not indicated otherwise, prefereably refers on the one hand to luminescent nanoparticles, especially with an emissive semiconductor dot core, e.g. CdSe, CdTe or the like) that is preferably (for example in order to increase the quantum yield of emission and/or to avoid photobleaching) capped with a thin shell of a higher band gap material, such as ZnS, CdS, ZnSe or the like; preferably, such particles have a size in the range from 0,5 to 50. more preferably (to obtain the desired luminescence properties) 1 to 10 nm, e.g. from 2 to 7 nm. These luminescent nanoparticles are therefore appropriate for luminescence based assay systems, e.g. using fluorescence in situ hybridisation (FISH) or other luminescence based methods. On the other hand, the term nanoparticles preferably refers also to nanoparticles (especially in the method described as second embodiment of the invention) with magnetic (especially ferro- or paramagnetic) properties and, to that purpose, comprise a ferro- or paramagnetic material, especially ferrite, magnetite, Pt/Fe alloys or magnetic rare earth based material (which may also show luminescence), such as Gadolinium. Dysprosium, Erbium or also Vanadium. These latter magnetic nanoparticles are, after using the methods described in the first, second or third embodiments according to the invention and subsequent derivatisation with biomolecules, of particular interest especially in the area of molecular imaging. Such magnetic particles may preferably have a size in the range from 0,5 to 80, more preferably 1 to 40 nm, e.g. from 5 to 30 nm.

The method according to the first embodiment of the present invention can be applied to nanoparticles which alreacly have hydroxy-groups on their surface, such as SiO₂ or ferric oxides or nanoparticles obtainable as described in the second embodiment of the invention, the method according to the subsequent second embodiment can be used to manufacture nanoparticles where these groups have to be applied.

In a second embodiment, the invention relates to a method for the manufacture of nanoparticles carrying a second type of coating exposing hydroxyl, mercapto or amino groups accessible from the outside of the nanoparticles carrying said coating, wherein nanoparticles are reacted in a solvent without active hydrogen in the presence of a multidentate ligand with three or more groups selected from the group consisting of hydroxy, amino and thio, with the proviso that at least two of these groups are amino or (preferably) thio.

In a more preferred version of this embodiment of the invention, first the nanoparticles are added to the solvent without active hydrogen and after that the multidentate ligand as defined above is added.

The method for applying hydroxy-groups to nanoparticles according to the second embodiment of the invention appears to correspond to a method previously published by Pathak et al. (Pathak S et al., 2001 J. Am. Chem. Soc., 123, pages 4103-4104.) in contrast to that method which employs reflux conditions, where it has not been possible by the present inventors to confirm the concept that the hydroxy groups of the multidentate ligand therein are those which react with biomolecules ― rather, the sequence of events described by Pathak et al. indicates that the binding biomolecules may replace the multidentate ligand therein instead of reacting with hydroxy groups thereof, corresponding to known methods of thiol replacement. Also, a stable dry preparation has not been described in the Pathak reference. In contrast, the present invention, especially working at lower temperatures than reflux and with particularly good results where dimethylsulfoxide is used as solvent, leads to products that enable the manufacture of dried preparations and/or where instead of a replacement of multidentate ligands rather a reaction with free mercapto, amino or preferably hydroxy groups takes place in the case of reaction with biomolecules.

A solvent without active hydrogen is preferably a solvent without hydrogen bound to N, S or O (e.g. in amino, imino, mercapto of hydroxy).

The dried preparation nanoparticles with the hydroxy group exposing coating can be prepared especially advantageously, because inter alia their inclination to agglomeration and the formation of aggregates and clumps is thus strongly diminished and the powder properties are especially advantageous, in an aprotic amphoteric or amphophilic solvent (the term solvent including both single solvents and solvent mixtures), preferably any one of the following solvents without active hydrogen: N,N-disubstituted C₁-C₇-alkanoylamides, such as N,N-formamide, especially N,N-dimethylformamide or N,N-dimethylacetamide, nitrites, such as C₁-C₇-alkylcyanides, especially acetonitrile, cyclic ethers with one ring oxygen, especially tetrahydrofurane, or preferably di-C₁-C₇-alkylsulfoxides, especially dimethylsulfoxide, or mixtures of two or more of these solvents;
or, where the reaction further (as described below as third embodiment of the invention) comprises as a next reaction step the crosslinking of the multidentate ligand is conducted as in the first embodiment of the invention, one or more of these solvents or a cyclic ether with more than one ring oxygen, e.g. dioxane.

For good reaction yield, the solvent or solvents used preferably have only a low water content (e.g. less than 0.5 %), more preferably they are practically free of water (anhydrous/absolute).

Most preferred, as this gives the least clumping or agglomeration and/or especially makes it possible to obtain a dry preparation with high stability and high dispersion status that can be redispersed conveniently, especially first using dimethylsulfoxide, is a solvent comprising, especially consisting of dirrethylsulfoxide as solvent.

The reaction can take place at various temperatures, but especially good results are obtained if the reaction takes place below the reflux temperature, e.g. preferably in the range from -80 ° C to 60 °C, but more preferably in a range from 0 to 40 °C, e.g. at ambient or room temperature.

A multidentate ligand with three or more groups selected from the group consisting of hydroxy, amino and thio, with the proviso that at least two of these groups are amino or (preferably) thio, is preferably a circular, linear and/or branched molecule with up to 16 carbon atoms, preferably with up to 6 carbon atoms, that carries at least two amino or thio groups and at least one fur:her amino, thio or preferably hydroxy group.

Preferably, the multidentate ligand carries at least two hydroxy and at least two mercapto groups. Most preferred is d thiothreitol (threo-1,4-dimercapto-2,3-butanediol).

Without being bound to this explanation solely, it is believed that the multidentate ligand forming the coating is chemisorbed via two or more groups selected from amino or especially thio to the nanoparticles used as starting material, white at least one amino or mercapto or preferably hydroxy group is exposed and accessible from the outside of the resulting coated nanoparticles for chemical derivatisation.

The nanoparticles used as starting materials in the second embodiment of the invention can also carry other adsorbed molecules, such as trialkylphosphines or their oxides, alcohols, such as n-butanol, or other reagents that remain adsorbed to their surface after their manufacture. These adsorbed molecules are then displaced, preferably replaced (partially or preferably practically completely) by the multidentate ligands used as the other starting material.

Preferably, in a further step the nanoparticles with the second type of coating are then isolated, e.g. by precipitation and optionally washing with a solvent of lower polarity, such as a di-C₁-C₇-alkyl ether, e.g. diethylether, and dried to form a dry preparation, especially in the form of an amorphous powdery material, e.g. under vacuum, especially by lyophilisafion, most preferably forming an amorphous lyophilisate. This nanoparticle preparation, especially the lyophilisate, is readily dispersible in polar media, such as water, buffer solutions and the like.

A third embodiment of the invention relates to a combination of the method of the first and the second embodiment of the invention, thus yielding a type of coating where the nanoparticles are coated by crosslinked multidentate ligands and carry hydroxy groups exposed to their outside.

As stated above, surprisingly the methods of the first, second and third embodiment allow for the manufacture of nanoparticles with exposed reactive hydroxyl; hydroxyl, amino and/or mercapto; or again hydroxyl groups, respectively, and result in highly dispersed coated nanoparticles that allow for easy handling.

In a fourth embodiment, the invention therefore relates to a coated nanoparticle preparation obtainable according to the method (especially the preferred variants thereof) of the second embodiment of the invention, especially in the form of a dry preparation, especially in the form of an amorphous lyophilisate.

In a fifth and sixth embodiment, the invention relates to a coated nanoparticle preparation obtainable according to the methods (especially the preferred variants thereof) of the first or of the third embodiment of the invention, especially in the form of a dry preparation, in particular in the form of an amorphous lyophilisate.

The coated nanoparticles according to the fourth, fifth and sixth embodiment of the invention can be stored for longer periods of time, preferably at lower temperatures, e.g. from -80 to 0°C, for example from -30 to -15 °C, but they can also be stored at higher temperatures for longer times, e.g. severaly days, for example at temperatures from 0 to 40 °C, e.g. in a refrigerator or at room temperature.

The invention, in a seventh embodiment, also relates to the further modification of nanoparticles by partially oxidizing the surface hydroxy-groups.

In an eighth embodiment, the invention also relates to re-dispersed ("dissolved") preparations of functionalised (e.g. luminescent, magnetic or other) nanoparticle preparations according to any one of the fourth, the fifth or the sixth embodiment of the invention, comprising the nanoparticles and at least one solvent.

In an ninth embodiment, the invention relates to the use of the coated nanoparticles according to the fourth, fifth or sixth embodiment or obtainable according to the seventh embodiment of the invention *in,* a method of using said nanoparticles in and/or a process *for* the manufacture of said nanoparticles coupled to biologically interesting components, thus allowing for the labelling of these molecules, e.g. for analytical, such as diagnostic, or for therapeutic (e.g. introducing luminescent nanoparticles with antibodies directed against tumor antigens, thus allowing inter alia for photodynamic therapy or the like, or of magnetic nanoparticles e.g. to keep nanoparticles loaded with drugs at a place of treatment in the body by applying magnetic fields) purposes. The coupling takes place by direct binding to the exposed hydroxy, (where present) mercapto or (where present) amino groups on the nanoparticles or preferably via a linker.

Direct dispersion (optionally first using a solvent, such as dimethyl suffoxide, of the coated nanoparticles, and then diluting with an appropriate solvent for the coupling reaction) and direct coupling or coupling via one or more further linkers of the nanoparticles according to the invention is possible in any common solvent, e.g. buffer. This makes the coated nanoparticles according to the invention to a universal tool for biolabelling.

Biologically interesting components can, for example, be selected from the group consisting of compounds, e.g. nucleotides, such as mono-, oligo- or polynucleotides, e.g. DNA or RNA, PNA, proteins or peptides, such as enzymes, receptor ligands, receptors, antibodies, ligand binding proteins, such as avidine, streptavidine, protein A, protein G, His-tags or the like, membrane proteins, protein complexes, or fragments or derivatives thereof, e.g. glutathione coupled peptides, glykosylated or fatty acid derivatives thereof, polysaccharides, lipids, liposomes, substrates for enzymes, lower molecular weight compounds, such as ligands, drugs, enzyme substrates, microorganisms, such as multicellular or preferably unicellular prokaryotes or eukaryotes, organelles, such as mitochondria, membrane vesicles, virus or virus parts, and the like. This enumeration is not intended to be limiting. Preferably, the binding of the biologically interesting component(s) takes place via a carboxy (without linker or via a linker) or (without linker or via a linker) an amino mercapto or hydroxy group present on the biologically active component.

Linkers are preferably molecular constitutents that are e.g. formed from coupling reagents or especially bi(or higher)-functional coupling agents. The person having skill in the art will be able to decide which linkers are, taking chemical and other aspects into consideration, are appropriate for a specific application and which are not.

The derivatisation, where the binding takes place via a carboxy group present on the molecule, can, for example, take place in the presence of coupling reagents that activate the carboxy group, such as *in situ,* for example dicyclohexylcarbodiimide/1-hydroxybenzotriazole (DCC/ HOBT); bis(2-oxo-3-oxazolidinyl)phosphinic chloride (BOPCI); O-(1,2-dihydro-2-oxo-1-pyridyl)-*N,N,N',N'*-tetramothyluronium tetrafluoroborate (TPTU); O-benzotriazol-1-yl)-N,N,N', N'-tetramethyluronium tetrafluoroborate (TBTU); (benzotriazol-1-yloxy)-tripyrrolidino-phosphonium-hexafluorophosphate (PyBOP), 1-(3-dimethylaminopropyl)-3-ethylcarbodiimide hydrochloride/hydroxybenzotriazole or/1-hydroxy-7-azabenzotriazole (EDC/HOBT or EDC/HOAt) or HOAt alone, or with (1-chloro-2-methyl-propenyl)-dimethylamine, or the like.

The reaction preferably takes place by reacting the nanoparticles according to the fourth, fifth or sixth embodiment of the invention with one or more at least and preferably bifunctional reagents that derivatise hydroxy, mercapto or amino groups, present (exposed) on the nanoparticles or on the biologically interesting component, so that subsequently a reaction between the reactants, that is, the (underivatized or derivatized) nanoparticle and the (derivatized or underivatized) biologically interesting component, can take place. The bifunctional reagents work as linkers between the nanoparticles and the biologically interesting components.

As at least bifunctional reagents, homo-bi- (or in a broader embodiment also tri-)functional reagents are preferred, for example, diisothiocyanates, especially 1,3-diisothiocyano-benzene, bis-succinimidyl derivatives, especially disuccinimidyl glutarate, dithiobis(succinimidoyl-propionate), disuccinimidoyl suberate, disuccinimidoyl tartrate, ethylen glycol-bis(succinimidylsuccinate) or bis(2-(succinimidyloxycarbonyloxy)ethyl)sulfone, methyl imidates, such as dimethyl pimelidate, dimethyl-3,3'-dithiobispropionimidate, dimethyl-adipimidate or dimethylsuberimidate (e.g. as hydrochloride salts), sulfosuccinimidoyl derivatives, such as 3,3'-dithiobis(sulfosuccinimidyl proprionate), disulfosuccinimidyl tartrate, ethylene glycol-bis(sulfosuccinimidylsuccinate), bis-(sulfosuccinimidoyl)-suberate or bis(2-(sulfosuccinimidooxycarbonyloxy)ethyl)sulfone, bis-carbodiimides, such as 1-ethyl-3-(3-dimethylaminopropyl)-carbodiimide hydrochloride, or 1,5-difluoro-2,4-dinitro-benzene.

For linking of sulfhydryl coupling group comprising reactants, especially sulfhydryl-reactive homobifunctional linkers can be used, e.g. bis-maleimides, such as dithio-bis-maleidoethane, bis-vinyl sulfones, such as 1,6-hexane-bis-vinylsulfone, 1,4-bis-maleimido-butane. 1,4-bis-maleimidyl-2,3-dihydroxybutane, bis-maleimidohexane, bis-maleimido-ethane, 1,8-bis-maleimidotriethylenglycol or 1,11-bis-maleimidotetraethylenglycol.

Of course, it is also possible (preferably where the hydroxy groups on the nanoparticles are cross-linked according to the fourth or with lower preference the sixth embodiment of the invention) to use hetero-bifunctional linker reagents, e.g. reagents that comprise one group that reacts with amino, hydroxy or mercapto on one of the reactants, and another group that is a disulfide that may subsequently be reduced to yield free mercapto groups (e.g. with dithio-threitol or the like) that may then be reacted with a mercapto group on the other reactant by mild oxidation.

Other possible hetero-bifunctional linkers are, e.g., linkers carrying one amino-, hydroxy- or mercapto-reactive group and a photoactivatable group. Here, first chemical reaction of the amino-, hydroxy- or mercapto-reactive group leads to modification of a reactant carrying such groups, and subsequently photoreaction can be used for the linking. Examples of such linkers are N-hydroxysuccinimidoyl-4-azido-salicylic acid, N-succinmidoyl-(4-azidophenyl)-1,3-dithiopropoinate, sulfosuccinimidoyl-2-(7-azido-4-methylcoumarin-3-acetamido)ethyl-1,3'-dithiopropionate, sulfosuccinimidoyl-2-(m-azido-o-nitrobenzamido)ethyl-1,3'-dithioproplonate, N-succinimidyl-6-(4'-azido-2'-nitro-phenylamino)hexanoate, sulfosuccinimidyl-2-(p-alfaazido-salicylamido)ethyl-1,3'-dithiopropionate, N-hydroxysulfosuccinimidoyl-4-azido-benzoate, sulfosuccinimidoyl(4-azidosalicylamino)hexanoate, sulfosuccinimidyl(4-azidophenylthio)propionate, sulfosuccinimidoyl-6-(4'-azido-2'-nitrophenylamino)hexanoate, n-(4-(p-azidosalicyl-amido)butyryl)-3'-(2'-pyridyldithio)propionamide, or bis-(beta-(-azidosalicylamido)ethyl)disulfide.

Further possible hetero-bifuncational linkers are, for example, linkers that comprise one amino- and one sulfide-reactive group or two amino-reactive groups, respecttively, for example succinimidoyl-maleinimid derivatives, such as succinimidoyl-butylphenyl-maleinimide, N-epsilon-maleimidocaproic acid, or N-( epsilon maleimidocaproic acid) hydrazide, N-( epsilon-maleimidocaproiyloxy) succinimide ester, N-( gammamaleimidobutyryloxy)succinimide ester, N-kappamaleimidoundecanoic acid or its N-hydrazide, succinimidyl-4-(N-maleimidomethyl)-cyclohexane-1-carboxy-(6-amidocaproate), succinimidyl-6-(3-(2-pyridyldithio)propionamido)-hexanoate, m-maleimidobenzoyl-N-hydroxysuccinimide, succinimidoyl-3-(bromoacetamido)-propionate, N-( epsilonmaleimidocaproyloxy)sulfosuccinimide ester, N-(gamma-maleimidobutyryloxy)sulfosuccinimide ester, sulfosuccinimidoyl-4(rho-maleimidophenyl)butyrate, sulfosuccinimidyl-6-(alfa-methyl-alfa-(2-pyridyldithio)toluamideo)hexanoate, N-(alfa-maleimidoacetoxy)succinimidate: ester or N-(beta-maleimidopropyloxy)succinimde ester; or in the case of two amino-reactive groups methyl-N-succinimidoyl adipate.

An example for sulfhydryl- and hydroxyl-reactive heterobifunctional linkers is N-(p-maleimidophenyl)isocyanate (PMPI).

An example for hydroxy- and amino reactive heterobifunctional linkers is 1,1'-carbonyl diimidazole.

The reaction between one reactant and the linkers and the other reactant can take place in any appropriate sequence that leads to linkage of the reactants and under customary conditions.

In one version of the ninth embodiment of the invention, the reaction takes place under such conditions that only a low number of the hydroxy or hydroxy, mercapto or amino groups accessible on the coated nanoparticles according to the invention is actually derivatized by the linker, e.g. by using only a low concentration of a coupling reagent, such as CDI, so that only a low share of these reactive groups, e.g. approximately on average 1 per nanoparticle, is actually reacted. For example, for this purpose a coupling reagent may be added in a molar ratio with regard to the number of available hydroxy, mercapto or amino groups on the nanoparticles in the range from 1 : 10 to 1 : 100 000 (meaning one mol of coupling reagent is used for 10 to 100 000 mol of hydroxy, mercapto and/or amino groups on the nanoparticles). However, in other cases it may be desirable to bind more than one biologically interesting component, so that then other ratios may be useful.

After redispersion, e.g. in DMSO, the hydroxy (or with lower preference amino or mercapto) groups on the particles surface of can be linked with bifunctional agents as mentioned above, like PMPI, CDI or others, and precipitated again. The resulting precipitate can then be dried, e.g. lyophilized, and can be stored, e.g. under the conditions mentioned above for the nanoparticles with free hydroxy, mercapto and/or amino groups. The material can then be coupled to biomolecules in any appropriate buffer at any desired later time point.

In a tenth embodiment, the invention also relates to (especially luminescent or magnetic) nanoparticles obtainable according to the first, second or third embodiment of the invention coupled to biologically interesting components, especially as obtainable according according to the ninth embodiment of the invention.

The invention also relates to any derivative or solution obtained from nanoparticles obtainable according to the first, second or third embodiment of tne invention or from nanoparticle preparations according to the fourth, fifth or sixth embodiment of the invention.

In contrast to published procedures, the derivatised nanoparticles can be yielded as dry preparations with the invented methods. Direct dissolution and coupling of these powders in all common buffers to almost any biomolecule make these powders a general tool for biolabelling.

### Examples

The following examples illustrate the invention without limiting its scope:

### Example 1. CdSe/ZnS nanoparticies coated with DTT and their coupling with streptavidine and the use of the streptavidine coupled nanoparticles

**Synthesis of DTT coated CdSe/ZnS-nanoparticies.** CdSe/ZnS nanocrystals are prepared according to a literature prccedure (e.g. Nann T, 2005 Chem. Comm., 13, pages 1735-1736, incorporated by reference herein regarding the description of the preparation and nanocrystals) or purchased from a commercial source. 5-7 mg CdSe/ZnS nanocrystals dispersed in 1 ml of chloroform are precipitated with methanol and washed for three times. Subsequently, they are redispersed in 1 ml of a 0.1-0.2 M solution of DTT in dimethylsulfoxide (DMSO) and stirred overnight at ambient temperature. Thereafter the particles are again precipitated with ether and redispersed in DMSO. CDI is added in a molar ratio of 1:1000 (CDI:DTT) in respect to the DTT in the solution used for redispersion above and incubated for 48 hours at 4 °C. The product is precipitated with ether and can be stored as dry amorphous material especially after lyphilisation.

**Derivatisation of DTT-coated nanoparticles with streptavidine and use of the streptavidine coupled nanoparticles:** The amorphous material is redispersed in 1 ml of an aqueous solution of 1 mg streptavidin and incubated at room temperature for 24 hours. The conjugate is purified by means of gel filtration. This purified nanocrystal-streptavidin conjugate is used for labelling biotinylated samples in a customary manner.

### Example 2: Experiment analogous to an Enzyme Linked Immunosorbant Assay:

1 ml of CdSe/ZnS core/Shell nanocrystals (NCs) are precipitated from a chloroform solution with methanol and and centrifuged. The precipitate is dissolved in 1 ml of 0.1 M dithiothreitol (DTT) in dry dimethylsulfox de (DMSO) and stirred for at least 24 h. The DTT modified NCs are precipitated by the addition of 1 ml diethyl ether and centrifugation. The precipitate is re-dispersed in 1 ml of 0.1 mM carbodiimide (CDI) in DMSO for activation. The solution is incubated for 48 h at 4 °C. After that, the modified and activated NCs are again centrifuged and washed several times with DMSO. After the last washing, the precipitate is dried in high vacuum. A solution of 0.1 mg streptavidine in 1 ml phosphate buffered saline (PBS) is added and vortexed. The resulting solution is incubated for further 48 h at 4 °C. To remove excessive streptavidine, the solution is dialyzed aga nst PBX for 24 h. During dialysis, PBS is exchanged four times and solution (A) is obtained.

A biotin tagged enzyme is incubated on a polystyrene plate. The plate is blocked with bovine serum albumine (BSA) and washed twice afterwards. The immobilisation of enzyme on plate and blocking follow standard techniques.

After washing, the binding reaction is carried out by incubation of dialyzed solution with the immobilised biotin tagged enzyme for 2h. The plate is washed three times with PBS, dried on air and read out with a fluorescence reader (excitation 458 nm). Rising fluorescence on the plate documents a successful biotin streptavidine binding reaction.

### Example 3: Nanoparticles with cross-linked coating

**Cross-linking of derivatised nanoparticles.** DTT-coated nanoparticles from Example 1 are cross-linked by means of tetraethoxysilane (TEOS). Therefore, 10 mg of DTT-coated nanoparticles are dispersed in 5 ml of anhydrous tetrahydrofuran (THF). TEOS is added in such an amount that theoretically a monolayer of SiO₂ is formed on the nanoparticles (calculated from the geometry of the particles) and stirred for at least 2 hours at room temperature. Subsequently, ammonia is added in an equimolar amount with respect to the TEOS, followed by stirring for further 2 hours. The cross-linked particles are obtained by centrifugation and washing with ethanol. Coupling to biomolecules is performed analogously to Example 3.

### Example 4: Synthesis of CdSe/ZnS/SiO₂-nanoparticles derivatised with streptavidin:

**Synthesis of CdSe/ZnS/SiO**_{**2**}**-Streptavidin.** Silica-coated CdSe/ZnS nanoparticles are prepared according to a previously published method (Nann T et al., 2004 Angew. Chem. Int. Ed., 43, pages 5393-5396, incorporated by reference herein regarding the description of the preparation and nanoparticles). Approximately 15 mg of silica-coated nanocrystals are dispersed in DMSO. CDI is added in a molar ratio of 10:1 in respect to the nanoparticles and incubated for 48 hours at 4 °C. The product is precipitated with ether and can be stored as dry powder. The powder is redispersed in 1 ml of an aqueous solution of 1 mg streptavidin and incubated at room temperature for 24 hours. The conjugate can be purified by means of gel filtration and used in the same manner as the conjugates from example 1.

### Example 5: CdSe/ZnS-nanoparticles and their coupling with oligonucleotides and the use of the oligonucleotide coupled nanoparticles

**Synthesis of CdSe/ZnS-Oligonucleotide conjugates.** 5-7 mg CdSe/ZnS nanocrystals dispersed in 1 ml of chloroform are treated as described in example 1 up t and including the DTT-coating. CDI is added in a molar ratio of 1:1000 with respect to the DTT and incubated for 48 hours at 4 °C. The product is precipitated with ether and can be stored as dry amorphous material. The dry amorphous material is re-dispersed in 1 ml of an aqueous solution of 1 mg oligonucleotides and incubated at room temperature for 24 hours. The conjugate is purified by means of gel filtration. Single stranded oligonucleotides are able to hybridise with complementary stands and can thus be used for labelling specific nucleotide sequences, e.g. in chromosomes or bacterial DNA.

### Example 6: Use of oligonucleotide-coupled CdSe/ZnS-nanoparticles

CdSe/ZnS core/shell nanocrystals (NCs) obtained according to Example 5 are precipitated from a solution (adjusted to an optical density of 2.0 at 488 nm excitation) in dimethylformamide (DMF).

The precipitation of the NCs is achieved by addition of 400 µl of 10 mg of N,N-dimethylaminopyridine in DMF to 150 µl of the latter solution, followed by centrifugation.

The precipitate is washed three times with DMF and centrifuged. After final centrifugation, the NCs are dissolved in 1 ml of thiol-terminated DNA. The solution is aged for 48 h and the salt concentration is brought to 0.2 M by the successive, portion-wise addition of NaCl during this period. The NC-DNA acduct is centrifuged and redispersed in water or phosphate buffered saline (PBS) for immediate further use.

## Claims

1. A method for the mariufacture of nanoparticles carrying a first type of coating with hydroxyl groups that are accessible from the outside of said nanoparticles, comprising reacting nanoparticles exposing hydroxyl, mercapto or amino groups accessible from the outside of the nanoparticles carrying said coating with a cross-linking reagent that is capable of cross-linking said hydroxy, mercapto or amino groups and itself carries hydroxy groups or precursor groups thereof and, if required, setting free the hydroxy groups from their procurer groups.

2. The method according to claim 1 wherein the cross-linking reagent is a silazane or a silane carrying three or more Si-bound halo and/or alkoxy groups, preferably a tetra-(C₁₋C₇-alkoxy)-silane.

3. The method according to any one of claims 1 or 2 where the reaction takes place in an alcohol and in the presence of a nitrogen base catalyst with at least one active hydrogen, preferably ammonia and/or primary or secondary amines.

4. The method according to claim 1 where the reaction takes place at from -80 °C to the reflux temperature of the reaction mixture, preferably in the range from 0 to 50 °C.

5. The method according to any one of claims 1 to 4 where the setting free of hydroxy groups from their precursor groups takes place by hydrolysis, preferably due to water present in the reaction mixture, so that the crosslinking and the hydrolysis take place in parallel.

6. The method according to any one of claims 1 to 5, comprising as an additional process step isolating the nanoparticles, especially by precipitation with a solvent of lower polarity, especially a di-C₁-C₇-alkylether, and drying to form a dry preparation, especially in the form of an amorphous powdery material, especially by administration of vacuum, more especially by lyophilisation, most preferably forming an amorphous lyophilisate.

7. A method according to any one of claims 1 to 6 where the nanoparticles are luminescent or magnetic.

8. A coated nanoparticle preparation obtainable according to the methods given in any one of claims 1 to 7.

9. The coated nanoparticle preparation according to claim 8 in the form of a dry preparation.

10. The coated nanoparticle preparation according to claim 8 in the form of a lyophilisate,

11. The use of coated nanopartides according obtainable according to the method in any one of claims 1 to 7 or especially of a coated nanoparticle preparation according to any one of claims 8 to 10 as a first reactant for the manufacture of said nanoparticles coupled to biologically interesting components as second reactant where first and second reactant are coupled directly or preferably via a linker to the hydroxy groups exposed on the nanoparticles.

12. A method for the manufacture of nanoparticles carrying a second type of coating exposing hydroxyl, mercapto or amino groups accessible from the outside of the nanoparticles carrying said coating, wherein nanoparticles are reacted in a solvent without active hydrogen in the presence of a multidentate ligand with three or more groups selected from the group consisting of hydroxy, amino and thio, with the proviso that at least two of these groups are amino or thio.

13. The method according to claim 12 where first the nanoparticles are added to the solvent without active hydrogen and after that the multidentate ligand is added.

14. The method according to any one of claims 12 or 13 wherein the reaction takes place in an aprotic amphoteric or amphophilic solvent, preferably an N,N-disubstituted C₁-C₇₋alkanoylamide, such as N,N-formamide, especially N,N-dimethylformamide or N,N-dimethylacetamide, a nitrile, such as C₁-C₇-alkylcyanide, especially acetonitrile, a cyclic ether with one ring oxygen, especially tetrahydrofurane, or preferably a di-C₁-C₇₋alkylsulfoxide, especially dimethylsulfoxide, or mixtures of two or more of these solvents.

15. The method according to any one of claims 12 to 14 wherein the reaction takes place at a temperature below the reflux temperature of the reaction mixture, preferably in the range from -80 °C to 60 °C, more preferably in a range from 10 to 40 °C, most preferably at ambient or room temperature.

16. The method according to any one of claims 12 to 15 wherein as solvent dimethylsulfoxide is used.

17. The method according to any one of claims 12 to 16 where as multidentate ligand preferably a circular, linear and/or branched molecule with up to 16 carbon atoms, preferably with up to 6 carbon atoms, that carries at least two amino or thio groups and at least one further amino, thio or preferably hydroxy group, more preferably that carries at least two hydroxy and at least two mercapto groups, most preferably dithiothreitol, is used.

18. The method according to any one of claims 12 to 17, comprising as an additional process step isolating the nanoparticles, especially by precipitation with a solvent of lower polarity, especially a di-C₁-C₇-alkylether, and drying to form a dry preparation, especially in the form of an amorphous powdery material, especially by administration of vacuum, more especially by lyophilisation, most preferably forming an amorphous lyophilisate.

19. A method according to any one of claims 1 to 6 where the nanoparticles are luminescent or magnetic.

20. A coated nanoparticle preparation obtainable according to the methods given in any one of claims 12 to 19.

21. The coated nanoparticle preparation according to claim 20 in the form of a dry preparation.

22. The coated nanoparticle preparation according to claim 20 in the form of a lyophilisate.

23. The use of coated nanoparticles according obtainable according to the method in any one of claims 12 to 19 cr especially of a coated nanoparticle preparation according to any one of claims 20 to 22 as a first reactant for the manufacture of said nanoparticies coupled to biologically interesting components as second reactant where first and second reactant are coupled directly or preferably via a linker to the hydroxy, mercapto and/or amino groups exposed on the nanoparticles.

24. A method for the manufacture of nanoparticles coated by crosslinked multidentate ligands and carrying hydroxy groups exposed to their outside, comprising first manufacturing nanoparticles exposing hydroxyl, mercapto or amino groups accessible from the outside of the nanoparticles carrying said coating according to a method according to a method any one of claims 12 to 19 and subsequently reacting these intermediate nanopartic es with a cross-linking reagent that is capable of cross-linking said hydroxy, mercapto or amino groups and itself carries hydroxy groups or precursors thereof using the method according to any one of claims 1 to 7.

25. A coated nanoparticle preparation obtainable according to the method given in claim 24, especially in the form of a dry preparation, more especially as lyophilisate.

26. The use of a coated nanoparticle preparation according to claim 25 as a first reactant for the manufacture of said nanoparticles coupled to biologically interesting components as second reactant where first and second reactant are coupled directly or preferably via a linker to the hydroxy groups exposed on the nanoparticles.

27. A redispersed preparation of a nanoparticle preparation according to any one of claims 8 to 10, 20 to 22 or 25, comprising said nanoparticle preparation and at least one solvent.
